# EUROPÄISCHE PATENTSCHRIFT

(11) **EP 0 931 087 B1**
(45) Veröffentlichungstag und Bekanntmachung des Hinweises auf die Patenterteilung: **03.04.2002**
(21) Anmeldenummer: 97944867.7
(22) Anmeldetag: 10.09.1997
(51) Int. Cl.: C07H 15/203, C07H 7/04, A61K 31/70

(54) **NEUE PYRANOSIDDERIVATE**
NOVEL PYRANOSIDE DERIVATIVES
NOUVEAUX DERIVES DE PYRANOSIDE

(30) Priorität: 12.09.1996 DE 19637123
(43) Veröffentlichungstag der Anmeldung: 28.07.1999
(73) Patentinhaber: Boehringer Ingelheim International GmbH, 55218 Ingelheim am Rhein (DE); Boehringer Ingelheim Pharma KG, 55218 Ingelheim am Rhein (DE)
(72) Erfinder: ANDERSKEWITZ, Ralf, D-55411 Bingen (DE); SCHROMM, Kurt, D-55218 Ingelheim am Rhein (DE); RENTH, Ernst-Otto, D-24105 Kiel (DE); BIRKE, Franz, D-55218 Ingelheim am Rhein (DE); JENNEWEIN, Hans, Michael, D-65193 Wiesbaden (DE); MEADE, Christopher, John, Montague, D-55411 Bingen (DE); DING, Andreas, D-55218 Ingelheim am Rhein (DE)
(74) Vertreter: Laudien, Dieter, Dr.
(86) Internationale Anmeldenummer: EP9704948
(87) Internationale Veröffentlichungsnummer: WO9811119

(56) Entgegenhaltungen:
- WO-A-93/16036
- WO-A-97/21670
- US-A- 5 246 965

## Beschreibung

Die vorliegende Erfindung betrifft neue Pyranosididderivate, Verfahren zu Ihrer Herstellung sowie ihre Verwendung als Arzneimittel. Die neuen Pyranosidderivate entsprechen der allgemeinen Formel I 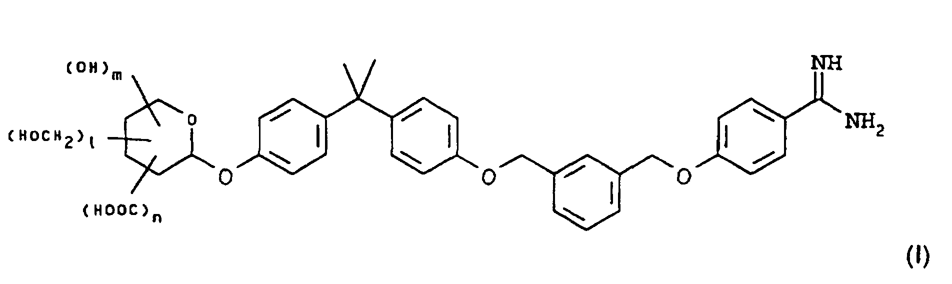 worin
l, m und n eine ganze Zahl 0, 1, 2, 3 und 4 bedeuten und l+m+n≤4
bedeuten - in Form ihrer Racemate, in enantiomerenreiner bzw. angereicherter Form, gegebenenfalls als Diastereomerenpaare und jeweils als freie Basen oder Salze, vorzugsweise mit physiologisch verträglichen Säuren, vorliegen.

Die erfindungsgemäßen Verbindungen sind potente LTB₄-Antagonisten. Insbesondere die Verbindung aus Beispiel 1 entsteht *in vivo* als Metabolit einer LTB₄-antagonistischen Verbindung und hat im Rezeptorbindungstest einen Kⱼ-Wert von 1,0 nM.

Wie gefunden wurde, zeichnen sich die Verbindungen der Formel I durch vielfältige Anwendungsmöglichkeiten auf therapeutischem Gebiet aus. Hervorzuheben sind solche Anwendungsmöglichkeiten, für welche die LTB₄-rezeptorantagonistischen Eigenschaften eine Rolle spielen. Hier sind insbesondere zu nennen: Arthritis, Asthma, chronische obstruktive Lungenerkrankungen, etwa chronische Bronchitis, Psoriasis, Colitis ulcerosa, durch nichtsteroidale Antiphlogistika induzierte Gastro- oder Enteropathie, cystische Fibrose, Alzheimer-Krankheit, Schock, Reperfusionsschäden/Ischämien, Atherosklerose, Multiple Sklerose.

Auch lassen sich mit den neuen Verbindungen Krankheiten oder Zustände behandeln, bei denen die Passage von Zellen aus dem Blut über das vaskuläre Endothelium in das Gewebe von Bedeutung ist (etwa Metastasis) oder Krankheiten und Zustände, bei denen die Kombination des LTB₄ oder eines anderen Moleküls (beispielsweise 12-HETE) mit dem LTB₄-Rezeptor einen Einfluß auf die Zell-Proliferation hat (etwa chronische myelozytische Leukämie).

Die neuen Verbindungen können auch in Kombination mit anderen Wirkstoffen angewendet werden, etwa solchen, die für dieselben Indikationen Verwendung finden, oder z.B. mit Antiallergika, Sekretolytika, β₂-Adrenergika, inhalativ anwendbaren Steroiden, Antihistaminika und/oder PAF-Antagonisten. Die Verabreichung kann topisch, oral, transdermal, nasal, parenteral oder inhalativ erfolgen.

Zur pharmakologischen und biochemischen Untersuchung der Wirkungsverhältnisse eigenen sich Tests, wie sie beispielsweise in der WO 93/16036, S. 15 bis 17 - auf die hier inhaltlich Bezug genommen wird - dargestellt sind.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 10 und 500 mg, vorzugsweise zwischen 20 und 250 mg. Bei inhalativer Anwendung werden dem Patienten zwischen etwa 0,5 und 25, vorzugsweise zwischen etwa 2 und 20 mg Wirkstoff zugeführt.

Inhalationslösungen enthalten im allgemeinen zwischen etwa 0,5 und 5 % Wirkstoff. Die neuen Verbindungen können in üblichen Zubereitungen verabreicht werden, etwa als Tabletten, Dragées, Kapseln, Oblaten, Pulver, Granulate, Lösungen, Emulsionen, Sirupe, Inhalationsaerosole, Salben, Suppositorien.

Die nachstehenden Beispiele zeigen einige Möglichkeiten für die Formulierung der Darreichungsformen:

### Formulierungsbeispiele

### 1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.

### 2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

### 3. Inhalationspulver

Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 33 45 722, auf die hiermit inhaltlich Bezug genommen wird, inhaliert.

Die Darstellung der erfindungsgemäßen Verbindungen erfolgt nach Methoden die an und für sich aus dem Stand der Technik bekannt sind. So können die Verbindungen der allgemeinen Formel I 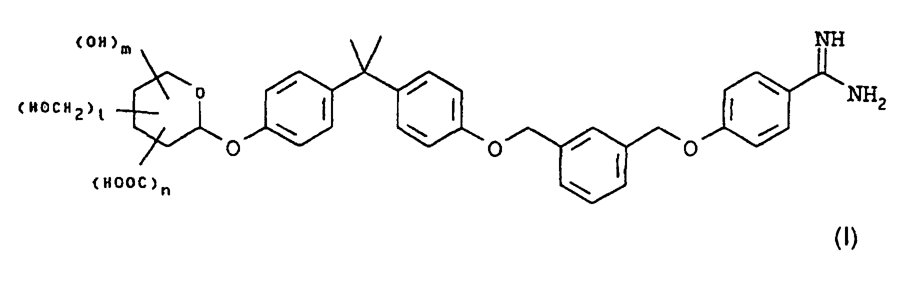 in der I, m und n die eingangs genannte Bedeutung haben, in der Art und Weise hergestellt werden, daß man 4-[[3-[[4-[1-(4-hydroxyphenyl)-1-methylethyl]phenoxy]methyl]phenyl]methoxy]-benzolcarboximidamid mit einem Glucosederivat der allgemeinen Formel II 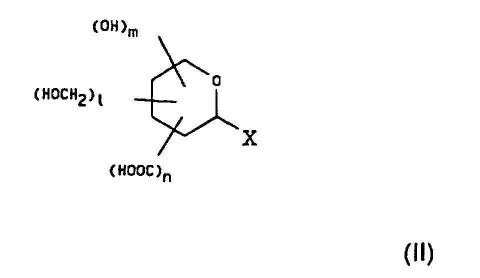 in dem im Falle n>0 die Carboxylgruppe gegebenenfalls in Form eines C₁-C₄-Alkylesters und die Hydroxygruppen in Form von Acylgruppen einer aliphatischen oder aromatischen Carbonsäure geschützt sind und X eine durch einen Phenolatsauerstoff substituierbare Austrittsgruppe bedeutet, mit dem entspechenden gewünschten Phenolat umsetzt und gegebenenfalls die Estergruppen verseift.

Die erfindungsgemäßen Verbindungen können darüber hinaus hergestellt werden aus einem gegebenenfalls geschützten Glucosederivat (II) und dem oben genannten Phenol mit Hilfe von basischen Schwermetallverbindungen wie z.B. Ag₂O oder CdCO₃ in inerten Solventien wie Toluol oder Dichlormethan. Gegebenenfalls wird das Produkt durch Verseifung der Schutzgruppen freigesetzt.

Die Verbindungen (I) können auch hergestellt werden aus Derivaten der Formel (II) und dem oben genannten Phenol mit Hilfe von Lewis-Säuren wie z.B. BF₃, AlCl₃, ZnCl₂, SnCl₄, oder TiCl₄, oder aus Alkoholat-derivaten dieser Lewissäuren in inerten Solventien wie Toluol, Dichlormethan etc.

Weiterhin können die erfindungsgemäßen Verbindungen dargestellt werden aus einem gegebenenfalls geschützten Derivat (II) mit X=OH und dem obengenannten Phenol mit Hilfe saurer Katalysatoren wie z.B. Methansulfonsäure oder Tetrafluoroborsäure oder mit Hilfe von Lewis-Säuren wie z.B. BF₃, AlCl₃, ZnCl₂, SnCl₄, oder TiCl₄, oder aus Alkoholat-derivaten dieser Lewissäuren in inerten Solventien wie aliphatischen, aromatischen, alkylsubstituierten Aromaten oder in einem halogenierten Kohlenwasserstoff - vorzugsweise in Toluol oder in Dichlormethan.

Dabei steht C₁-C₄-Alkyl im Sinne der o.a. Herstellungsverfahren im allgemeinen für einen verzweigten oder unverzweigten Kohlenwasserstoffrest mit 1 bis 4 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt: Methyl, Ethyl, Propyl, 1-Methylethyl (Isopropyl), n-Butyl, 1-Methylpropyl, 2-Methylpropyl und 1,1-Dimethylethyl.

Die erfindungsgemäßen Verbindungen sind ausgehend von aus dem Stand der Technik bekannten Verbindungen u.a. nach den in den folgenden Beispielen beschriebenen Verfahren herstellbar. Verschiedenartige, andere Ausgestaltungen der Verfahren werden für den Fachmann aus der vorliegenden Beschreibung ersichtlich. Es wird jedoch ausdrücklich darauf hingewiesen, daß diese Beispiele und die diesen zugeordnete Beschreibung lediglich zum Zweck der Erläuterung vorgesehen und nicht als Einschränkung der Erfindung anzusehen sind.

### Beispiele

### Beispiel 1

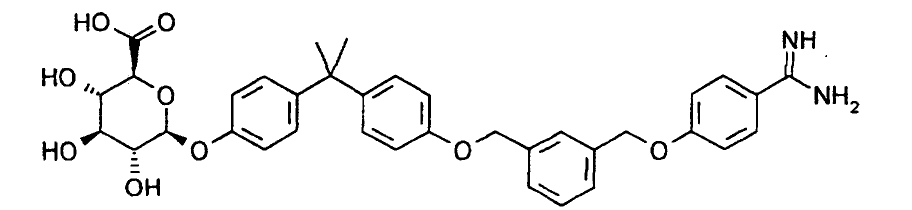

### 4-[[3-[[4-[1-(4-hydroxyphenyl)-1-methylethyl]phenoxy]methyl]phenyl]methoxy]-benzolcarboximidamid-O-glucuronid

Zu einer Lösung von 37,8 g 4-[[3-[[4-[1-(4-hydroxyphenyl)-1-methylethyl]phenoxy]methyl]phenyl]methoxy]-benzolcarboximidamid in 1000 ml Dimethoxyethan gibt man eine 30%ige Lösung von Natriummethylat in Methanol und läßt 10 min. rühren. Nach Abkühlen auf -10°C gibt man 32,4 g Acetobromo-α-D-glucuronsäuremethylester hinzu und läßt 48 h bei Raumtemperatur rühren. Nach Zugabe einer Lösung von 3,5 g LiOH in 100 ml Wasser läßt man weitere 48 h rühren und destilliert das Lösungsmittel bei Raumtemperatur im Vak. ab. Der Rückstand wird in 200 ml Laufmittel H¹) gelöst und die sich abscheidende wäßrige Phase abgetrennt. Die Substanz wird mit Hilfe präparativer Dünnschichtplatten chromatographiert, nach Extraktion mit Methanol isoliert und mit Wasser kristallisiert. Ausbeute: 5,3 g (Fp. 205°C (Zers.)).
¹⁾ 36 Teile n-Butanol, 15 Teile Ameisensäure, 15 Teile Wasser, 15 Teile Aceton, 5 Teile Chloroform werden zusammengegeben, gut geschüttelt, und nach 3 Tagen wird die abgeschiedene Wasserphase abgetrennt.

### Beispiel 2

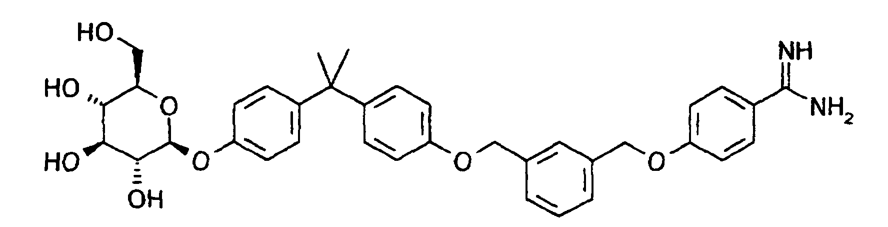

### 4-[[3-[[4-[1-(4-hydroxyphenyl)-1-methylethyl]phenoxy]methyl]phenyl]methoxy]-benzolcarboximidamid-O-glucose

Die Verbindung wird nach dem Verfahren aus Beipiel 1 aus 4-[[3-[[4-[1-(4-hydroxyphenyl)-1-methylethyl]phenoxy]methyl]phenyl]methoxy]-benzolcarboximidamid und Tetraacetylbromglucose hergestellt.

## Patentansprüche

1. Pyranosidderivate der allgemeinen Formel I 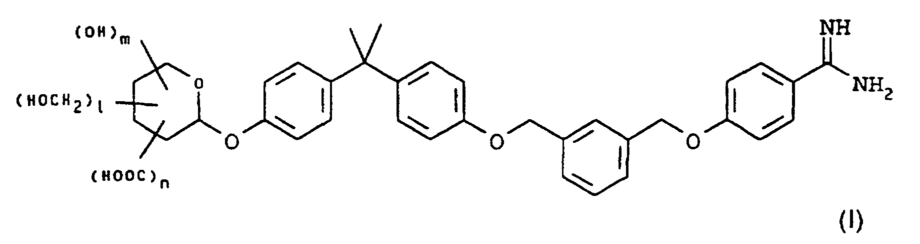 worin
l, m und n eine ganze Zahl 0, 1, 2, 3 und 4 bedeuten und l+m+n≤4 bedeuten - in Form ihrer Racemate, in enantiomerenreiner bzw. angereicherter Form, gegebenenfalls als Diastereomerenpaare und jeweils als freie Basen oder Salze, vorzugsweise mit physiologisch verträglichen Säuren.

2. 4-[[3-[[4-[1-(4-hydroxyphenyl)-1 methylethyl]phenoxy]methyl]phenyl]methoxy]-benzolcarboximidamid-O-glucuronid.

3. 4-[[3-[[4-[1-(4-hydroxyphenyl)-1-methylethyl]phenoxy]methyl]phenyl]methoxy]-benzolcarboximidamid-O-glucose.

4. Verfahren zur Herstellung von Verbindungen der allgemeinen Formel I 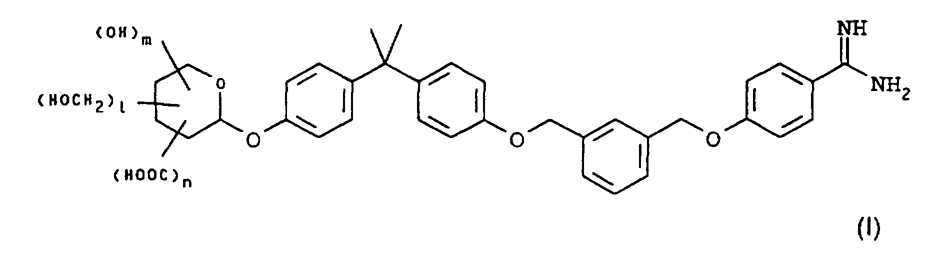 in der l, m und n die in Anpruch 1 genannte Bedeutung haben, **dadurch gekennzeichnet, daß** man 4-[[3-[[4-[1-(4-hydroxyphenyl)-1-methylethyl]phenoxy]methyl]phenyl]methoxy]-benzolcarboximidamid mit einem Glucosederivat der allgemeinen Formel II 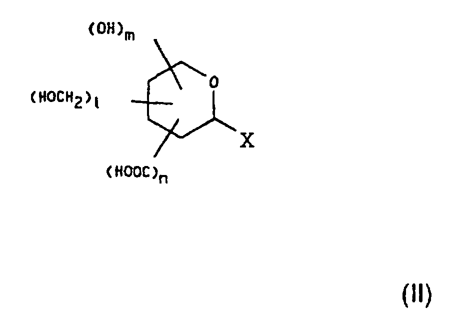 in dem im Falle n>0 die Carboxylgruppe gegebenenfalls in Form eines C₁-C₄-Alkylesters und die Hydroxylgruppen in Form von Acylgruppen mit einer aliphatischen oder aromatischen Carbonsäure geschützt sind und X eine durch einen Phenolatsauerstoff substituierbare Austrittsgruppe bedeutet, als Phenolat umsetzt und gegebenenfalls die Estergruppen verseift.

5. Verfahren nach Anspruch 4, **dadurch gekennzeichnet, daß** die Umsetzung in Gegenwart von einer sauer reagierenden Katalysatoren bzw. einer Lewis-Säure durchgeführt wird.

6. Verfahren nach Anspruch 5, **dadurch gekennzeichnet, daß** der sauer reagierende Katalysator Methansulfonsäure oder Tetrafluorborsäure ist.

7. Verfahren nach Anspruch 5, **dadurch gekennzeichnet, daß** die Lewis-Säure BF₃, AlCl₃, ZnCl₂, SnCl₄, oder TiCl₄ ist oder ein Alkoholat dieser Lewis-Säuren ist.

8. Verfahren nach Anspruch 4, **dadurch gekennzeichnet, daß** die Umsetzung in Gegenwart einer basisch reagierenden Übergangsmetallverbindung durchgeführt wird.

9. Verfahren nach Anspruch 8, **dadurch gekennzeichnet, daß** die Übergangsmetallverbindung Ag₂O oder CdCO₃ ist.

10. Verfahren nach einem der Ansprüche 4 bis 9, **dadurch gekennzeichnet, daß** als Reaktionsmedium ein aliphatischer oder aromatischer Kohlenwasserstoff, ein alkylsubstituierter Aromat oder ein halogenierter Kohlenwasserstoff eingesetzt wird.

11. Verfahren nach Anspruch 10, dadruch gekennzeichnet, daß als Lösungsmittel Toluol oder Dichlormethan eingesetzt wird.

12. Pharmazeutische Zubereitung, **gekennzeichnet durch** einen Gehalt an einer Verbindung nach einem der Ansprüche 1 bis 3 sowie deren pharmakologisch akzeptierbaren Säureadditionssalzen neben üblichen Hilfs- und Trägerstoffen.

13. Verwendung von Verbindungen nach einem der Ansprüche 1 bis 3 zur Herstellung eines Arzneimittel.

14. Verwendung von Verbindungen nach Anspruch 13 zur Herstellung eines Arzneimittels mit LTB₄-antagonistischer Wirkung.

15. Verwendung von Verbindungen der allgemeinen Formel 1, deren Stereoisomere sowie deren Säureadditionssalze zur Herstellung eines Arzneimittels zur therapeutischen Behandlung von Arthiritis, Asthma, chronischer obstruktiver Lungenerkrankung wie chronischer Bronchitis, Psoriasis, Colitis ulcerosa, durch nichtsteroidale Antiphlogistika induzierter Gastro- oder Enteropathie, cystischer Fibrose, Alzheimer-Krankheit, Schock, Reperfusionsschäden/Ischämien, Atherosklerose, multipler Sklerose.

## Claims

1. Pyranoside derivatives of general formula I 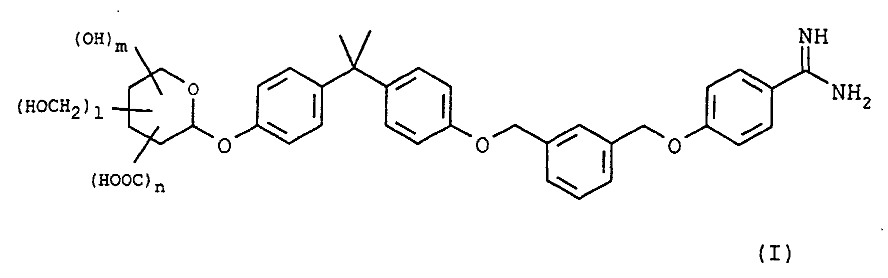 wherein
l, m and n denote an integer chosen from 0, 1, 2, 3 or 4, and l+m+n≤4
- in the form of their racemates, in enantiomerically pure or concentrated form, optionally as pairs of diastereomers and in each case in the form of free bases or salts, preferably with physiologically acceptable acids.

2. 4-[[3-[[4-[1-(4-hydroxyphenyl)-1-methylethyl]-phenoxy]methyl]phenyl]methoxy]-benzenecarboximidamide-O-glucuronide.

3. 4-[[3-[[4-[1-(4-hydroxyphenyl)-1-methylethyl]-phenoxy]methyl]phenyl]methoxy]-benzenecarboximidamide-O-glucose.

4. Process for preparing compounds of general formula I 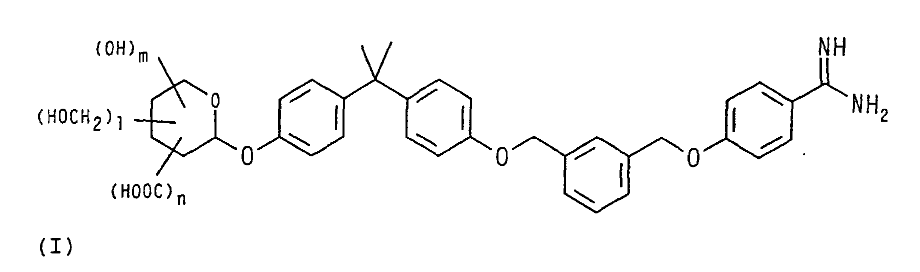 wherein l, m and n are defined as in claim 1,
**characterised in that** 4-[[3-[[4-[1-(4-hydroxyphenyl)-1-methylethyl]phenoxy]methyl]phenyl]methoxy]-benzenecarboximidamide is reacted with a glucose derivative of general formula II 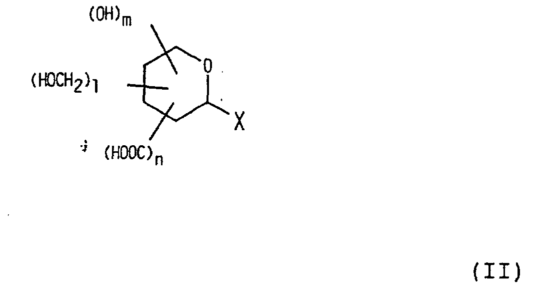 wherein, if n>0, the carboxyl group may optionally be in the form of a C1-4-alkylester and the hydroxyl groups in the form of acyl groups are protected with an aliphatic or aromatic carboxylic acid and X denotes a leaving group which may be substituted by a phenoxide oxygen, and optionally the ester groups are saponified.

5. Process according to claim 4, **characterised in that** the reaction is carried out in the presence of an acidically reacting catalyst or a Lewis acid.

6. Process according to claim 5, **characterised in that** the acidically reacting catalyst is methanesulphonic acid or tetrafluoroboric acid.

7. Process according to claim 5, **characterised in that** the Lewis acid is BF₃, AlCl₃, ZnCl₂, SnCl₄ or TiCl₄ or an alkoxide of these Lewis acids.

8. Process according to claim 4, **characterised in that** the reaction is carried out in the presence of a basically reacting transition metal compound.

9. Process according to claim 8, **characterised in that** the transition metal compound is Ag₂O or CdCO₃.

10. Process according to one of claims 4 to 9, **characterised in that** an aliphatic or aromatic hydrocarbon, an alkyl-substituted aromatic or a halogenated hydrocarbon is used as the reaction medium.

11. Process according to claim 10, **characterised in that** toluene or dichloromethane is used as solvent.

12. Pharmaceutical preparation, **characterised in that** it contains a compound according to one of claims 1 to 3 and the pharmaceutically acceptable acid addition salts thereof together with conventional excipients and/or carriers.

13. Use of compounds according to one of claims 1 to 3 for preparing a pharmaceutical composition.

14. Use of compounds according to claim 13 for preparing a pharmaceutical composition having an LTB₄-antagonistic activity.

15. Use of compounds of general formula I, the stereoisomers thereof and the acid addition salts thereof for preparing a pharmaceutical composition for the therapeutic treatment of arthritis, asthma, chronic obstructive pulmonary disease such as chronic bronchitis, psoriasis, ulcerative colitis, gastropathy or enteropathy induced by nonsteroidal antiinflammatories, cystic fibrosis, Alzheimer's disease, shock, reperfusion damage/ischaemia, atherosclerosis or multiple sclerosis.

## Revendications

1. Dérivés de pyranosides de formule générale I 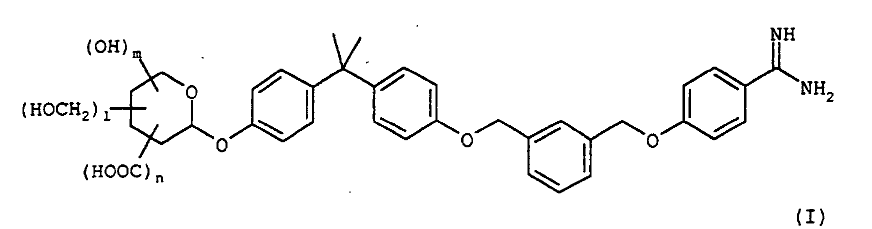 où
l, m et n représentent un nombre entier 0, 1, 2, 3 et 4 et l+m+n ≤ 4, sous forme de leurs racémates, sous forme énantiomériquement pure ou enrichie, éventuellement sous forme de paires de diastéréoisomères et dans chaque cas sous forme de bases libres ou de sels, de préférence avec des acides physiologiquement acceptables.

2. 4-[[3-[[4-[1-(4-hydroxyphényl)-1-méthyléthyl]phénoxy]méthyl]phényl]méthoxy]benzènecarbox-imidamide-O-glucuronide.

3. 4-[[3-[[4-[1-(4-hydroxyphényl)-1-méthyléthyl]phénoxy]méthyl]phényl]méthoxy]benzènecarboximidamide-O-glucose.

4. Procédé de préparation de composés de formule générale I 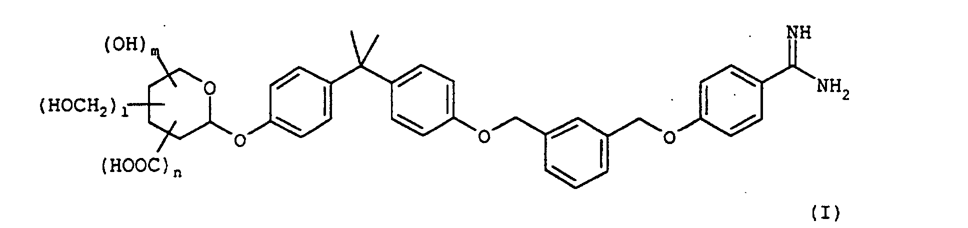 où l, m et n ont la signification citée dans la revendication 1, **caractérisé en ce que** l'on fait réagir le 4-[[3-[[4-[1-(4-hydroxyphényl)-1-méthyléthyl]-phénoxy]méthyl]phényl]méthoxy]benzènecarboximidamide sous forme de phénolate avec un dérivé du glucose de formule générale II 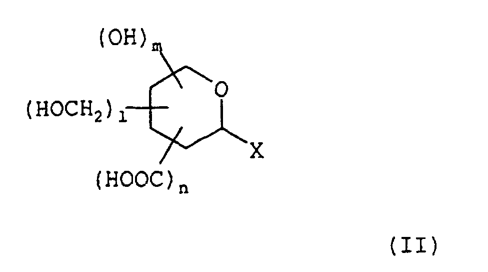 où, dans le cas où n > 0, le groupe carboxyle est éventuellement protégé sous forme d'un ester d'alkyle en C₁-C₄ et les groupes hydroxyle sont éventuellement protégés sous forme de groupes acyle avec un acide carboxylique aliphatique ou aromatique et X représente un groupe partant substituable par un oxygène de phénolate, et on saponifie éventuellement les groupes ester.

5. Procédé selon la revendication 4 **caractérisé en ce que** la réaction est conduite en présence d'un catalyseur à réaction acide ou d'un acide de Lewis.

6. Procédé selon la revendication 5 **caractérisé en ce que** le catalyseur à réaction acide est l'acide méthanesulfonique ou l'acide tétrafluoroborique.

7. Procédé selon la revendication 5 **caractérisé en ce que** l'acide de Lewis est BF₃, AlCl₃, ZnCl₂, SnCl₄ ou TiCl₄ ou un alcoolate de ces acides de Lewis.

8. Procédé selon la revendication 4 **caractérisé en ce que** la réaction est conduite en présence d'un composé de métal de transition à réaction basique.

9. Procédé selon la revendication 8 **caractérisé en ce que** le composé de métal de transition est Ag₂O ou CdCO₃.

10. Procédé selon l'une des revendications 4 à 9 **caractérisé en ce que** l'on utilise comme milieu réactionnel un hydrocarbure aliphatique ou aromatique, un composé aromatique alkyl-substitué ou un hydrocarbure halogéné.

11. Procédé selon la revendication 10 **caractérisé en ce que** l'on utilise le toluène ou le dichlorométhane comme solvant.

12. Préparation pharmaceutique **caractérisée par** une teneur en un composé selon l'une des revendications 1 à 3 ainsi que ses sels d'addition d'acide pharmacologiquement acceptables, outre des adjuvants et supports habituels.

13. Utilisation de composés selon l'une des revendications 1 à 3 pour la production d'un médicament.

14. Utilisation de composés selon la revendication 13 pour la production d'un médicament à effet antagoniste de LTB₄.

15. Utilisation de composés de formule générale I, de leurs stéréoisomères ainsi que de leurs sels d'addition d'acide pour la production d'un médicament pour le traitement thérapeutique de l'arthrite, de l'asthme, d'une maladie pulmonaire obstructive chronique comme la bronchite chronique, du psoriasis, de la colite ulcéreuse, d'une gastro- ou entéropathie induite par des antiphlogistiques non stéroldiens, de la fibrose kystique, de la maladie d'Alzheimer, du choc, des lésions de reperfusion/ischémies, de l'athérosclérose, de la sclérose en plaques.
